## Europäisches Patentamt
## European Patent Office
## Office européen des brevets

(19)

(11) Veröffentlichungsnummer: **0 308 772 B1**

(12) **EUROPÄISCHE PATENTSCHRIFT**

(45) Veröffentlichungstag der Patentschrift: **23.10.91**

(51) Int. Cl.5: **C07D 249/16**

(21) Anmeldenummer: **88114914.0**

(22) Anmeldetag: **13.09.88**

(54) Verfahren zur Herstellung von mit aromatischen Systemen kondensierten Triazolen.

(30) Priorität: **24.09.87 DE 3732169**

(43) Veröffentlichungstag der Anmeldung:
**29.03.89 Patentblatt 89/13**

(45) Bekanntmachung des Hinweises auf die
Patenterteilung:
**23.10.91 Patentblatt 91/43**

(84) Benannte Vertragsstaaten:
**CH DE FR LI**

(56) Entgegenhaltungen:

EP-A- 0 062 864    DE-A- 2 822 506
US-A- 3 564 001    US-A- 3 637 514
US-A- 4 158 660    US-A- 4 170 521
US-A- 4 299 965    US-A- 4 363 914

CHEMICAL ABSTRACTS, Band 95, Nr. 9,
31.August 1981, Columbus, Ohio, USA; OT-
SUKA CHEMICAL CO. Ltd.: "Purification of
benzotriazoles", Seite 790, Spalte 2,
Zusammenfassung-Nr. 80969x

(73) Patentinhaber: **BAYER AG**

**W-5090 Leverkusen 1 Bayerwerk(DE)**

(72) Erfinder: **Schnegg, Ulrich, Dr.**
**Nietzschestrasse 8**
**W-5090 Leverkusen(DE)**
Erfinder: **Bormann, Ulrich, Dr.**
**Auf dem Feldchen 9**
**W-5163 Langerwehe(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von mit aromatischen Systemen kondensierten Triazolen durch Umsetzung von o-Arylen-diaminen mit Nitriten und anschließender destillativer Reinigung.

Mit aromatischen systemen kondensierte Triazole sind wertvolle Korrosionsinhibitoren, insbesondere für Kupferoberflächen, können aber auch als Zwischenprodukte für zahlreiche Synthesen eingesetzt werden.

Es ist bereits bekannt, o-Phenylendiamin und o-Diaminotoluole in überschüssiger Essigsäure mit Natriumnitrit umzusetzen (DE-OS 23 51 595, Äquivalent zu US 3.970.667; US 3.732.239; US 3.564.001). Weiterhin ist es bekannt, anstelle des Natriumnitrits einen niederen Alkylester der salpetrigen Säure einzusetzen (DE-OS 28 22 506); in diesem Fall wird in einem aprotischen Lösungsmittel, wie einem Kohlenwasserstoff, oder in einem protischen Lösungsmittel, wie in einem Alkohol oder Etheralkohol, der auch geringe Mengen Wasser enthalten kann, gearbeitet. Zum Start der Diazotierung und der Ringschlußreaktion zum Triazol ist eine katalytische Menge Säure, beispielsweise einer Carbonsäure, erforderlich. Anstelle der Carbonsäure als Reaktionsstarter ist auch das sauer wirkende Benzotriazol bzw. Tolyltriazol eingesetzt worden (EP 62 864; EP 75 459).

Eine besondere Beachtung fand stets die Reinigung des erhaltenen kondensierten Triazols. So wird im Verfahren der obengenannten DE-OS 23 51 595 der Reaktionsansatz ohne Zwischenisolierung des Triazols mit wäßriger Natronlauge alkalisch gestellt und mehrmals einer Klärfiltration unterworfen; nach dem erneuten Ansäuern mit Salpetersäure wurde das ausgefällte Triazole als solches isoliert. In der DE-OS 28 22 506 wird nach dem Abdestillieren flüchtiger Anteile das Reaktionsgemisch angeimpft und das Triazol durch Kristallisation gewonnen. US 3.732.239 zeigt, daß beim Einsatz von rohem Diaminotoluol vor Beginn der Diazotierung und Ringschlußreaktion eine aufwendige Reinigung durch Destillation notwendig ist. Aus dem Reaktionsgemisch, welches Wasser und Essigsäure enthält, wird nach der Umsetzung mit wäßrigem Natriumnitrit das Tolyltriazol mit Chloroform extrahiert. Nach dem Abdestillieren des Chloroforms wird das Tolyltriazol umkristallisiert, beispielsweise aus Benzol.

Es ist jedoch auch versucht worden, nach einer Diazotierung mit Natriumnitrit in einem Wasser/Essigsäure-Reaktionsmedium das Benzotriazol zunächst als Öl abzutrennen, mit Wasser zu waschen und schließlich das Benzotriazol selbst unter vermindertem Druck zu destillieren. Da diese einfache Vakuumdestillation offenbar nicht völlig befriedigte, wurde die Reinigung von Benzotriazol und Tolyltriazol durch Vakuumdestillation in Gegenwart geringer Mengen an Formaldehyd verfeinert (US 4.170.521).

Die destillative Reinigung brachte anscheinend keine ausreichende Reinheit und Farbstabilität der kondensierten Triazole, so daß in EP 745 459, offenbar zur Vermeidung einer nicht befriedigenden Destillation, ein noch anderes Verfahren gezeigt wird, bei dem in $C_6$-$C_{10}$-Alkanolen als dem Reaktionsmedium mit Alkylnitriten gearbeitet wird und das erhaltene Triazol mit wäßriger Natronlauge aus diesem Reaktionsmedium extrahiert wird.

Es wurde nun überraschend gefunden, daß man trotz der nicht völlig befriedigenden Ergebnisse des Standes der Technik eine destillative Reinigung von kondensierten Triazolen vornehmen kann, wenn man die Destillation in Gegenwart eines alkalisch reagierenden Stoffes durchführt.

Die Erfindung betrifft daher ein Verfahren zur Herstellung von mit aromatischen Systemen kondensierten Triazolen durch Umsetzung von rohen oder gereinigten o-Arylen-diaminen mit Nitriten und anschließender destillativer Reinigung, das dadurch gekennzeichnet ist, daß die Destillation des kondensierten Triazols in Gegenwart einer alkalisch reagierenden, nicht flüchtigen (Erd)alkali-Verbindung in einer Menge von 0,1-20 Gew.-%, bezogen auf das Gewicht des kondensierten Triazols, durchgeführt wird.

Als alkalisch reagierende, nicht flüchtige (Erd)-alkali-Verbindungen kommen in Frage; alkalisch reagierende Verbindungen des Lithiums, Natriums, Kaliums, Rubidiums, Caesiums, Magnesiums, Calciums, Strontiums oder Bariums, in bevorzugter Weise alkalisch reagierende Verbindungen des Natriums, Kaliums, Calciums oder Magnesiums, in besonders bevorzugter Weise alkalisch reagierende Natrium-oder Calcium-Verbindungen und in ganz besonders bevorzugter Weise alkalisch reagierende Natrium-Verbindungen.

Als solche Verbindungen seien die Hydroxide, Oxide, Carbonate, Hydrogencarbonate und Salze anderer schwacher Säuren genannt. In bevorzugter Weise kommen die Carbonate, Hydroxide und Oxide von (Erd)alkali-Metallen in Frage, in bevorzugter Weise die Hydroxide oder Oxide und in besonders bevorzugter Weise die Hydroxide.

Diese alkalisch reagierenden Verbindungen können dem zu reinigenden kondensierten Triazol vor Eintritt in die Destillationsapparatur zugesetzt werden oder auch simultan mit dem zu reinigenden Triazol gemeinsam mit diesem oder getrennt von ihm in die Destillationsapparatur eingebracht werden. Hierzu kann die alkalisch reagierende Verbindung als solche oder in Lösung, beispielsweise in wäßriger Lösung eingesetzt werden. Eine bevorzugte Dosierungsform ist die der wasserlöslichen Hydroxide von (Erd)alkali-Metallen in Form einer wäßrigen Lösung. Solche wäßrigen Lösungen haben beispielsweise eine Konzentration von 2-70 Gew.-%, bevorzugt 10-60 Gew.-%, besonders bevorzugt 30-55 Gew.-% an Hydroxid, bezogen auf die gesamte wäßrige Lösung.

Der alkalisch reagierende Stoff wird in einer Menge von 0,01-20 Gew.-%, bevorzugt 0,05-10 Gew.-%, besonders bevorzugt 0,1-1 Gew.-%, bezogen auf das Gewicht das kondensierten Triazols, eingesetzt.

Als o-Arylen-diamine kommen beispielsweise o-Phenylendiamin, o-Naphthylendiamin oder o-Pyridylendiamin, bevorzugt o-Phenylendiamin, in Frage. Solche o-Arylendiamin können ein- oder mehrfach mit niederem Alkyl, beispielsweise mit 1-4 C-Atomen, wie Methyl, Ethyl, Propyl, Isopropyl, Butyl oder Isobutyl, mit Halogen, wie Fluor, Chlor oder Brom, mit Nitro, mit niederem Alkoxy, beispielsweise mit 1-4 C-Atomen, wie Methoxy, Ethoxy, Propoxy oder Butoxy, substituiert sein. Von den genannten Substituenten können 1-3, bevorzugt 1-2, besonders bevorzugt 1 Substituent auf dem o-Arylen-diamin vorhanden sein. Daneben ist das nicht substituierte o-Arylendiamin bevorzugt. In besonders bevorzugter Weise seien o-Phenylendiamin, Nitrophenylendiamin, Hydroxyphenylendiamin, Chlorphenylendiamin, 2,3-Diamino-toluol und 3,4-Diamino-toluol genannt.

Diese o-Arylen-diamine können sowohl in reiner als auch in roher Form, wie sie in technischen Herstellungsverfahren anfallen, eingesetzt werden.

Als Nitrite kommen Alkalinitrit, beispielsweise Natriumnitrit, oder niedere Alkylester der salpetrigen Säure, in bevorzugter Weise Alkylester der salpetrigen Säure, in Betracht. Solche Alkylester haben beispielsweise 1-6 C-Atome, bevorzugt 1-2 C-Atome, besonders bevorzugt 1 C-Atom; Beispiele sind Methylnitrit, Ethylnitrit, Propylnitrit, Isopropylnitrit, Butylnitrit, Isobutylnitrit, Amylnitrit, Isoamylnitrit und Hexylnitrit. Als Reaktionsmedium kommt ein wäßrig-saures Medium, beispielsweise unter Einbeziehung von Essigsäure, Propionsäure oder Buttersäure, Mineralsäuren oder ein organisches Medium aus Kohlenwasserstoffen oder Alkoholen oder Etheralkoholen in Betracht. In bevorzugter Weise wird ein organisches Lösungsmittel, besonders bevorzugt ein Alkohol als Reaktionsmedium benutzt. Solche Alkohole können 1-6 C-Atome, bevorzugt 1-3 C-Atome, besonders bevorzugt 1 C-Atom, haben, wie Methanol, Ethanol, Propanol, Isopropanol, Butanol, Isobutanol, Amylalkohol oder Hexanol. In bevorzugter Weise wird die Kombination der Verwendung eines $C_1$-$C_6$-Alkylnitrits, bevorzugt eines $C_1$-$C_2$-Alkylnitrits, besonders bevorzugt des Methylnitrits mit einem $C_1$-$C_6$-Alkanol, bevorzugt $C_1$-$C_3$-Alkanol, bevorzugt Methanol, angewandt.

Wenn vollständiger Umsatz erreicht werden soll, dann wird das Nitrit im allgemeinen in einer Menge von 1-1,2 Mol, bevorzugt 1,0-1,1 Mol, pro Mol des o-Arylendiamins eingesetzt.

Für den Fall, daß mit einem Alkalinitrit gearbeitet wird, wird im allgemeinen in Gegenwart einer Säure, beispielsweise in Gegenwart von Essigsäure oder Mineralsäuren, gearbeitet. Für den Fall, daß mit einem Alkylnitrit gearbeitet wird, kann in Gegenwart von Säure, beispielsweise Essigsäure oder Mineralsäuren, gearbeitet werden; anstelle der Essigsäure kann jedoch auch das herzustellende kondensierte Triazol als sauer reagierender Starter für die Diazotierung und Ringschlußreaktion eingesetzt werden. Für den Fall, daß mit Alkylnitrit in einem alkoholischen Lösungsmittel gearbeitet wird, ist es jedoch eine bevorzugte Variante des erfindungsgemäßen Verfahrens, daß auf jeden sauren Starter, etwa der genannten Art, verzichtet werden kann.

Das erfindungsgemäße Verfahren wird im allgemeinen bei einer Temperatur von -20 bis +100°C, bevorzugt von 20-80°C, durchgeführt. Die Durchführung bei Normaldruck ist die allgemeine Verfahrensvariante. Eine Durchführung bei Überdruck oder Unterdruck ist jedoch ebenso möglich.

Das erfindungsgemäße Verfahren kann sowohl absatzweise als auch kontinuierlich durchgeführt werden. In einer bevorzugten Verfahrensvariante wird die Herstellung der kondensierten Triazole in kontinuierlicher Weise, beispielsweise in einer Blasensäulen-Apparatur, ausgeführt. Die Menge des alkoholischen Reaktionsmediums wird hierbei mit etwa 50-200 ml Alkanol pro 100 g o-Arylendiamin angesetzt. Das Alkylnitrit als bevorzugtes Umsetzungsmittel wird im Falle des Methylnitrits beispielsweise in einem Methylnitrit-Generator aus Natriumnitrit, Methanol und Schwefelsäure hergestellt und für den bevorzugten Umsatz in einer ein- oder mehrstufigen Blasensäulen-Apparatur in etwa äquimolaren Mengen eingesetzt (wobei ein geringer molarer Überschuß im obengenannten Rahmen zulässig ist). Für den Fall einer absatzweisen Umsetzung wird gasförmiges Methylnitrit im allgemeinen bis zur Sättigung in die alkoholische Lösung des o-Arylen-diamins eingeleitet.

Zur Aufarbeitung eines Reaktionsansatzes, der beispielsweise unter Verwendung eines alkoholischen Lösungsmittels und eines Alkylnitrits erhalten worden war, wird im allgemeinen das alkoholische Lösungsmittel, gegebenenfalls gemeinsam mit etwa mitverwendetem Wasser, abdestilliert und der Rückstand einer solchen ersten Destillation gemeinsam mit einem alkalisch reagierenden Stoff oder nach Zusatz eines solchen zum rohen kondensierten Triazol einer geeigneten weiteren Destillationseinrichtung, beispielsweise einem Dünnschichtverdampfer oder einem Fallstromverdampfer, zugeführt. Zur Vergleichmäßigung der Benetzung der wärmeübertragenden Oberfläche einer solchen Destillationseinrichtung kann das rohe kondensierte Triazol mit einem polymeren oder oligomeren Wachs wie hochmolekulare Polydiole oder Paraffine versetzt werden, welches als nicht destillierbarer Rückstand wieder gewonnen wird.

Das erfindungsgemäß herstellbare kondensierte Triazol behält seine klare, vielfach schwach gelbliche Farbe über eine lange Zeit, während kondensierte Triazole, die nach einem Verfahren des Standes der Technik hergestellt worden waren, rasch eine Verfärbung zeigen und den Glanz des frisch destillierten Materials verlieren.

Ein besonderer Vorteil des erfindungsgemäßen Verfahren liegt darin, daß im allgemeinen mit rohen, besonders preisgünstigen Diaminen ein farbstabiles, hochreines Endprodukt erhalten wird.

Beispiel 1: Benzotriazol (zum Vergleich)

108 g frisch destilliertes 99,8 %iges o-Phenylendiamin wurden in 125 ml Methanol suspendiert und auf 50°C erwärmt. Bei dieser Temperatur wurde gasförmiges Methylnitrit, hergestellt aus Natriumnitritlösung (25 %ig), Methanol und Schwefelsäure (48 %ig), eingeleitet. Die Einleitgeschwindigkeit war so bemessen, daß kein Abgas dem Reaktionskolben entströmte. Sobald das Reaktionsgemisch kein Gas mehr aufnahm (ca. 2 Stunden) wurde eine Probe entnommen und auf vollständigen Umsatz überprüft. Anschließend wurde aus dem Reaktionsgemisch zunächst Methanol, dann Wasser destilliert. Danach wurde unter Zusatz von Polywachs (Polydiol 1550) als Gleitmittel über einen Dünnschichtverdampfer bei 4 mbar und einer Heizmitteltemperatur von 220°C kontinuierlich destilliert. Benzotriazol destillierte bei ca. 165°C und 4 mbar. Ein solch frisch destilliertes Benzotriazol ist nach gaschromatographischer Analyse 99,9 %ig im Gehalt. Es weist eine schwach hellgelbe Färbung auf. Nach mehrwöchigem Stehen ist die hellgelbe Farbe zu einem hellen beigefarbenen Farbton verblaßt.

Beispiel 2: Benzotriazol

Das Beispiel wurde durchgeführt wie das Beispiel 1. Statt frisch destilliertem hochreinem o-Phenylendiamin wurde jedoch ein rohes o-Phenylendiamin mit einem Gehalt von 98,8 % (Rest: p-Phenylendiamin, Anilin, Chloraniline und Unbekannte) eingesetzt und wie beschrieben mit Methylnitrit umgesetzt. Nach Abdestillieren von Methanol wurde ca. 1 Gew.-% an 50 %iger Natronlauge, bezogen auf das rohe Benzotriazol, zugegeben. Anschließend erfolgte die Destillation wie in Beispiel 1. Das erhaltene Benzotriazol (99,9 %ig) war hellgelb. Auch nach monatelangem Stehen blieb die klare Färbung vollends erhalten.

Beispiel 3: Benzotriazol (zum Vergleich)

Beispiel 2 wurde wiederholt, jedoch ohne Zusatz von Natronlauge. Das frisch destillierte Benzotriazol (99,9 %ig) war hellgelb, verfärbte sich aber innerhalb weniger Tage bis Wochen über beige nach graugrün.

Beispiel 4: Tolyltriazol

244 g rohes o-Toluylendiamin (Isomerengemisch aus 2,3-und 3,4-Diaminotoluol) wurden in 210 ml Methanol gelöst und bei 50°C wie in Beispiel 1 mit Methylnitrit umgesetzt. Nach Zugabe von ca. 1 Gew.-% 50 %iger Natronlauge wurde wie in Beispiel 1 destilliert. Die Kopftemperatur betrug bei 4 mbar ca. 175°C. Das destillierte Tolyltriazol war 99,7 %ig. Die Farbe war hellgelb und blieb über Monate unverändert.

Beispiel 5: Tolyltriazol

In einer kontinuierlich betriebenen Blasensäule wurde am Fuß der Blasensäule (80 mm ⌀) ein Gemisch aus 6 Teilen o-Toluylendiamin und 4 Teilen Methanol (enthielt ca. 10 % Wasser) mit einer Geschwindigkeit von 10 kg/h eingepumpt. Ebenso am Fuß der Blasensäule wurde über eine Ringdüse ca. 3,0 kg/h gasförmiges Methylnitrit, hergestellt in einem kontinuierlich betriebenen Gasgenerator aus Methanol, Natriumnitritlösung und wäßriger Schwefelsäure, eingeleitet. Das Abgas der Blasensäule wurde über einen Wäscher mit o-Toluylendiamin in Methanol entsorgt. Das Reaktionsgemisch aus dem Wäscher kann der Blasensäule zugeführt werden. Nach Verlassen der Blasensäule wurde aus dem Reaktionsgemisch kontinuierlich zunächst das Methanol, dann nach Zugabe von ca. 1 Gew.-% Natronlauge (50 %ig) das Wasser und schließlich das gebildete Tolyltriazol abdestilliert. Das Produkt war hellgelb und farbstabil.

**Patentansprüche**

1. Verfahren zur Herstellung von mit aromatischen Systemen kondensierten Triazolen durch Umsetzung von o-Arylen-diaminen mit Nitriten und anschließender destillativer Reinigung, dadurch gekennzeichnet, daß die Destillation des kondensierten Triazols in Gegenwart einer alkalisch reagierenden, nicht flüchtigen (Erd)alkali-Verbindung in einer Menge von 0,1-20 Gew.-%, bezogen auf das Gewicht des kondensierten Triazols, durchgeführt wird.

2. Verfahren nach Anspruch 1, dadurch gekennzeichnet, daß in Gegenwart einer alkalisch reagierenden Natrium-, Kalium-, Calcium- oder Magnesium-Verbindung, gearbeitet wird.

3. Verfahren nach Anspruch 1, dadurch gekennzeichnet, daß als alkalisch reagierender Stoff ein Hydroxid oder Oxid eines Alkali- oder Erdalkali-Metalls, eingesetzt wird.

4. Verfahren nach Anspruch 3, dadurch gekennzeichnet, daß ein wasserlösliches Hydroxid in Form einer 2-70 gew.-%igen wäßrigen Lösung eingesetzt wird.

5. Verfahren nach Anspruch 4, dadurch gekennzeichnet, daß eine 10-60 gew.-%ige wäßrige Lösung des Hydroxids eingesetzt wird.

6. Verfahren nach Anspruch 1, dadurch gekennzeichnet, daß 0,2-10 Gew.-%, der alkalisch reagierenden Verbindung, bezogen auf das Gewicht des kondensierten Triazols, eingesetzt werden.

7. Verfahren nach Anspruch 1, dadurch gekennzeichnet, daß o-Phenylendiamin oder o-Toluylendiamin umgesetzt wird.

8. Verfahren nach Anspruch 1, dadurch gekennzeichnet, daß als Nitrit ein $C_1$-$C_6$-Alkylnitrit eingesetzt wird und in einem $C_1$-$C_6$-Alkanol als Reaktionsmedium gearbeitet wird.

9. Verfahren nach Anspruch 1, dadurch gekennzeichnet, daß die Umsetzung in Abwesenheit einer sauer reagierenden Verbindung gestartet wird.

10. Verfahren nach Anspruch 1, dadurch gekennzeichnet, daß die Umsetzung kontinuierlich durchgeführt wird.

## Claims

1. Process for preparing triazoles fused with aromatic systems by reaction of o-arylene-diamines with nitrites and subsequent purification by distillation, characterised in that the distillation of the fused triazole is carried out in the presence of an alkaline, non-volatile alkali(ne earth) metal compound in an amount of 0.1-20% by weight, based on the weight of the fused triazole.

2. Process according to Claim 1, characterised in that the reaction is carried out in the presence of an alkaline sodium compound, potassium compound, calcium compound or magnesium compound.

3. Process according to Claim 1, characterised in that the alkaline substance used is a hydroxide or oxide of an alkali or alkaline earth metal.

4. Process according to Claim 3, characterised in that a water-soluble hydroxide is used in the form of a 2-70% strength by weight aqueous solution.

5. Process according to Claim 4, characterised in that a 10-60% strength by weight aqueous solution of the hydroxide is used.

6. Process according to Claim 1, characterised in that 0.2-10% by weight of the alkaline compound, based on the weight of the fused triazole, are used.

7. Process according to Claim 1, characterised in that o-phenylenediamine or o-toluylenediamine is reacted.

8. Process according to Claim 1, characterised in that the nitrite used is a $C_1$-$C_6$-alkyl nitrite and the reaction is carried out in a $C_1$-$C_6$-alkanol as the reaction medium.

9. Process according to Claim 1, characterised in that the reaction is initiated in the absence of an acidic compound.

10. Process according to Claim 1, characterised in that the reaction is carried out continuously.

## Revendications

1. Procédé de production de triazoles condensés avec des systèmes aromatiques par réaction de o-arylènediamines avec des nitrites, suivie d'une purification par distillation, caractérisé en ce que la distillation du triazole condensé est conduite en présence d'un composé alcalin (alcalino-terreux) non volatil, à réaction alcaline, en une quantité de 0,1 à 20 % en poids par rapport au poids du triazole condensé.

2. Procédé suivant la revendication 1, caractérisé en ce qu'on opère en présence d'un composé à réaction alcaline de sodium, de potassium, de calcium ou de magnésium.

3. Procédé suivant la revendication 1, caractérisé en ce qu'on utilise comme substance à réaction alcaline un hydroxyde ou oxyde d'un métal alcalin ou alcalino-terreux.

4. Procédé suivant la revendication 3, caractérisé en ce qu'on utilise un hydroxyde hydrosoluble sous forme d'une solution aqueuse à 2-70 % en poids.

5. Procédé suivant la revendication 4, caractérisé en ce qu'on utilise une solution aqueuse à 10-60 % en poids de l'hydroxyde.

6. Procédé suivant la revendication 1, caractérisé en ce qu'on utilise 0,2 à 10 % en poids du composé à réaction alcaline par rapport au poids de triazole condensé.

7. Procédé suivant la revendication 1, caractérisé en ce qu'on fait réagir la o-phénylènediamine ou la o-toluènediamine.

8. Procédé suivant la revendication 1, caractérisé en ce qu'on utilise comme nitrite un nitrite d'alkyle en $C_1$ à $C_6$ et on opère dans un alcanol en $C_1$ à $C_6$ comme milieu réactionnel.

9. Procédé suivant la revendication 1, caractérisé en ce que la réaction est amorcée en l'absence d'un composé à réaction acide.

10. Procédé suivant la revendication 1, caractérisé en ce qu'on conduit la réaction en continu.